(19) Europäisches Patentamt
European Patent Office
Office européen des brevets

(11) Veröffentlichungsnummer: **0 481 300 A2**

(12) **EUROPÄISCHE PATENTANMELDUNG**

(21) Anmeldenummer: **91116916.7**

(22) Anmeldetag: **04.10.91**

(51) Int. Cl.5: **C07D 211/58**, C07D 401/12, C07D 401/14, C08K 5/3435, C08K 5/3492

(30) Priorität: **16.10.90 DE 4032744**

(43) Veröffentlichungstag der Anmeldung: **22.04.92 Patentblatt 92/17**

(84) Benannte Vertragsstaaten: **BE CH DE ES FR GB IT LI NL**

(71) Anmelder: **BASF Aktiengesellschaft**
**Carl-Bosch-Strasse 38**
**W-6700 Ludwigshafen(DE)**

(72) Erfinder: **Aumueller, Alexander, Dr.**
**An der Marlach 5**
**W-6705 Deidesheim(DE)**
Erfinder: **Trauth, Hubert**
**Milanstrasse 6**
**W-6724 Dudenhofen(DE)**

(54) **Polyalkylpiperidinderivate und deren Verwendung als Lichtschutzmittel.**

(57) Polyalkylpiperidin-Derivate der allgemeinen Formel I

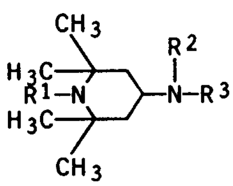

I

in der

$R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,

$R^2$ für einen Rest der Formel (a)

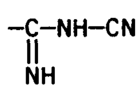

(a)

für einen Rest der Formel (b)

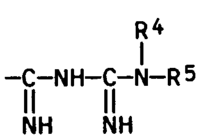

(b)

EP 0 481 300 A2

oder einen Rest der Formel (c)

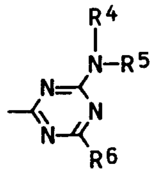

(c)

steht, wobei

$R^4$ und $R^5$ Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl, durch Ethersauerstoff- oder Stickstoff-Atome unterbrochenes $C_3$- bis $C_{22}$-Alkyl, welches zusätzlich Hydroxylgruppen tragen kann, Phenyl, das unsubstituiert oder ein- bis vierfach durch $C_1$- bis $C_{12}$-Alkyl, $C_3$- bis $C_{12}$-Cycloalkyl, $C_1$- bis $C_{12}$-Alkoxy, Chlor, Brom, $C_1$- bis $C_{12}$-Alkylamino, $C_1$- bis $C_{12}$-Dialkylamino, Phenyl, Benzyl, Hydroxyl oder Cyano substituiert sein kann, oder einen 5- oder 6-gliedrigen aromatischen Heterocyclus bedeuten, mit der Maßgabe, daß mindestens einer der Reste $R^4$ oder $R^5$ ein aromatischer Rest ist, und

$R^6$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl oder einen Rest der Formel

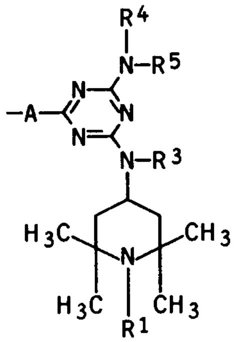

worin

A $C_2$- bis $C_{22}$-Alkylen, $C_8$- bis $C_{22}$-Cycloalkylen, $C_8$- bis $C_{16}$-Phenylalkylken, Phenylen oder durch Ethersauerstoff-Atome, Stickstoff-Atome oder 5- oder 6-gliedrige Heterocyclen unterbrochenes $C_4$- bis $C_{30}$-Alkylen bedeutet,

bezeichnet, und

$R^3$ Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl oder Bicycloalkyl, durch Ethersauerstoff- oder Stickstoff-Atome unterbrochenes $C_4$- bis $C_{22}$-Alkyl, welches zusätzlich Hydroxylgruppen tragen kann, Phenyl, welches durch ein bis drei Methyl- oder Carbo-$C_1$-$C_{12}$-alkoxy-Gruppen substituiert sein kann, heterocyclische Reste enthaltendes $C_1$-bis $C_{22}$-Alkyl, einen Rest der Formel

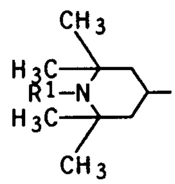

oder bei Vorliegen des Restes (a) für $R^2$ einen Rest der Formel

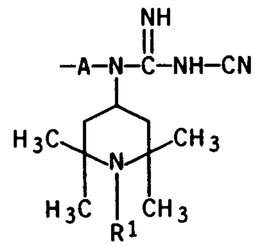

oder bei Vorliegen des Restes (b) für $R^2$ einen Rest der Formel

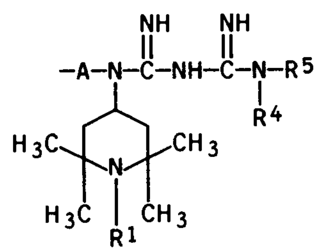

oder bei Vorliegen des Restes (c) für $R^2$ einen Rest der Formel

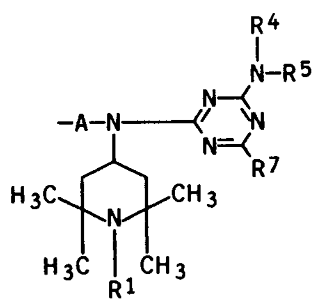

wobei $R^7$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl bedeutet,

bezeichnet,
sowie Tautomere und Säureadditionssalze der Verbindungen I.

Die Verbindungen I dienen als Lichtschutzmittel und Stabilisatoren für organisches Material.

Die vorliegende Erfindung betrifft neue Polyalkylpiperidin-Derivate der allgemeinen Formel I

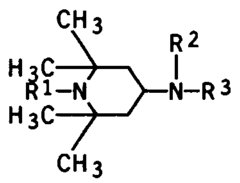

I

in der

$R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,

$R^2$ für einen Rest der Formel (a)

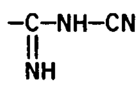

(a)

für einen Rest der Formel (b)

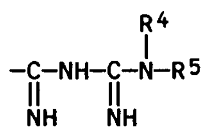

(b)

oder für einen Rest der Formel (c)

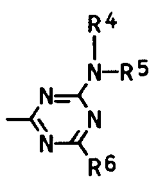

(c)

steht, wobei

$R^4$ und $R^5$ Wasserstoff, $C_1$- $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl, durch Ethersauerstoff- oder Stickstoff-Atome unterbrochenes $C_3$- bis $C_{22}$-Alkyl, welches zusätzlich Hydroxylgruppen tragen kann, Phenyl, das unsubstituiert oder ein- bis vierfach durch $C_1$- bis $C_{12}$-Alkyl, $C_3$- bis $C_{12}$-Cycloalkyl, $C_1$- bis $C_{12}$-Alkoxy, Chlor, Brom, $C_1$- bis $C_{12}$-Alkylamino, $C_1$- bis $C_{12}$-Dialkylamino, Phenyl, Benzyl, Hydroxyl oder Cyano substituiert sein kann, oder einen 5- oder 6-gliedrigen aromatischen Heterocyclus bedeuten, mit der Maßgabe, daß mindestens einer der Reste $R^4$ oder $R^5$ ein aromatischer Rest ist, und

$R^6$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl oder einen Rest der Formel

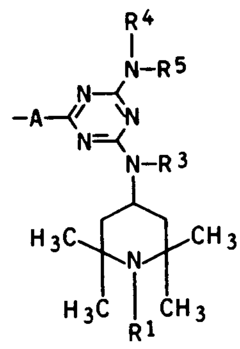

worin

A $C_2$- bis $C_{22}$-Alkylen, $C_8$- bis $C_{22}$-Cycloalkylen, $C_8$- bis $C_{16}$-Phenylalkylken, Phenylen oder durch Ethersauerstoff-Atome, Stickstoff-Atome oder 5- oder 6-gliedrige Heterocyclen unterbrochenes $C_4$- bis $C_{30}$-Alkylen bedeutet,

bezeichnet, und

$R^3$ Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl oder Bicycloalkyl, durch Ethersauerstoff- oder Stickstoff-Atome unterbrochenes $C_4$- bis $C_{22}$-Alkyl, welches zusätzlich Hydroxylgruppen tragen kann, Phenyl, welches durch ein bis drei Methyl- oder Carbo-$C_1$-$C_{12}$-alkoxy-Gruppen substituiert sein kann, heterocyclische Reste enthaltendes $C_1$- bis $C_{22}$-Alkyl, einen Rest der Formel

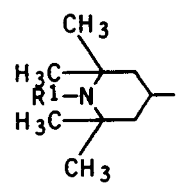

oder bei Vorliegen des Restes (a) für $R^2$ einen Rest der Formel

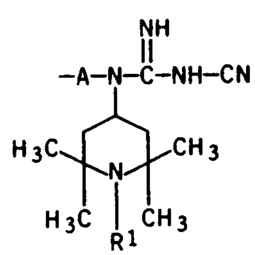

oder bei Vorliegen des Restes (b) für $R^2$ einen Rest der Formel

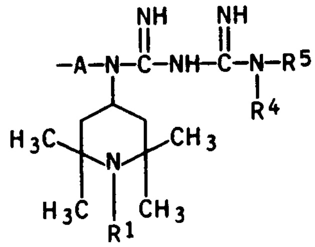

oder bei Vorliegen des Restes (c) für $R^2$ einen Rest der Formel

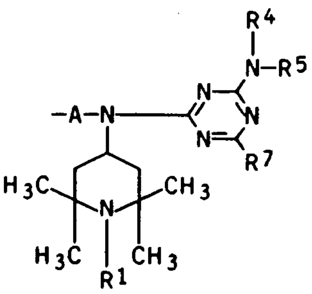

wobei $R^7$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl bedeutet,

bezeichnet,
sowie Tautomere und Säureadditionssalze der Verbindungen I.

Die DE-A 33 04 266 (1) betrifft Piperidin-Derivate der allgemeinen Formel II

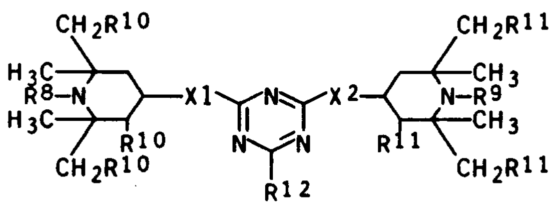

II

in der $R^8$ bis $R^{12}$ Wasserstoff oder Kohlenwasserstoffreste bezeichnen und $X^1$ und $X^2$ sauerstoff- oder stickstoffhaltige Brückenglieder bedeuten. Die Verbindungen II werden als Stabilisatoren für Polymere empfohlen.

Organisches Material, insbesondere Kunststoffe und Lacke, wird bekanntermaßen sehr schnell, vor allem durch Einwirkung von Licht, zerstört. Diese Zerstörung zeigt sich üblicherweise in Vergilbung, Verfärbung, Rißbildung oder Versprödung des Materials. Mit den bisher verwendeten Lichtschutzmitteln und Stabilisatoren konnte kein zufriedenstellender Schutz gegen die Zerstörung von organischem Material durch Licht, Sauerstoff und Wärme erzielt werden.

Aufgabe der vorliegenden Erfindung war es daher, Lichtschutzmittel bzw. Stabilisatoren bereitzustellen, die einen wirkungsvollen Schutz für organisches Material mit sich bringen.

Demgemäß wurden die eingangs definierten Polyalkylpiperidin-Derivate I gefunden.

Für $R^1$ kommen neben Wasserstoff vor allem Methyl, Ethyl, Propyl, Butyl, Pentyl, Hexyl, Benzyl, $\beta$-Phenylethyl, $\gamma$-Phenylpropyl, o-, m- oder p-Methylbenzyl, Allyl, Acetyl, Propionyl, Butanoyl, Pentanoyl, Benzoyl, Cyanmethyl, $\beta$-Hydroxyethyl, $\beta$-Aminoethyl und Hydroxyl sowie das Oxyl-Radikal in Betracht. Davon werden Methyl, Acetyl, Cyanomethyl, $\beta$-Aminoethyl und insbesondere Wasserstoff besonders bevorzugt.

Bei den Verbindungen I mit einem Rest der Formel (a) für $R^2$ handelt es sich um Cyanguanidin-Derivate, bei solchen mit einem Rest der Formel (b) um Biguanid-Derivate und bei solchen mit einem Rest der Formel (c) um Triazin-Derivate.

Für $R^3$ sind neben Wasserstoff z.B. zu nennen:

- geradkettiges und verzweigtes $C_1$- bis $C_{22}$-Alkyl, wie Methyl, Ethyl, n- und i-Propyl, n- und i-Butyl, n- und i-Pentyl, Hexyl, Octyl, Decyl, Dodecyl, Tetradecyl, Hexadecyl, Octadecyl, Pivalyl, 3,3-Dimethylbutyl-2, Neopentyl, 4-Methylpentyl-2 und 2-Ethylhexyl;
- $C_3$- bis $C_{22}$-Alkenyl wie Allyl, Butenyl, Pentenyl und Oleyl;
- $C_3$- bis $C_{12}$-Cycloalkyl wie Cyclopropyl, Cyclobutyl, Cyclopentyl, Cyclohexyl, Methylcyclohexyl,

Cycloheptyl, Cyclooctyl, Cyclododecyl und Bicycloheptyl, von denen Cyclopentyl und vor allem Cyclohexyl bevorzugt werden;

- durch Ethersauerstoff- oder Stickstoff-Atome unterbrochenes $C_4$- bis $C_{22}$-Alkyl, welches zusätzlich Hydroxylgruppen tragen kann, wie $-(CH_2)_3N(CH_3)_2$, $-(CH_2)_3N(C_2H_5)_2$, $-(CH_2)_3-OCH_3$, $-(CH_2)_3-O-CH-(CH_3)_2$, $-(CH_2)_2-O-(CH_2)_2-OH$, $-CH_2-(CH_2)_2-CH_2-N(CH_3)_2$, $-(CH_2)_2-N[CH(CH_3)_2]_2$, $-(CH_2)_2-N(C_2H_5)_2$, $-(CH_2)_2-N(CH_3)_2$, $-(CH_2)_2-OCH_3$ und $-(CH_2)_2OCH_2CH_3$;
- gegebenenfalls substituiertes $C_7$- bis $C_{22}$-Phenyl- und Diphenylalkyl wie Benzyl, Methoxybenzyl, Methylbenzyl, Ethylbenzyl, Isopropylbenzyl, Trimethylbenzyl, Fluorbenzyl, Chlorbenzyl, Methylendioxybenzyl, $\beta$-Phenylethyl, $\gamma$-Phenylpropyl und $\delta$-Phenylbutyl, Dimethylaminobenzyl, Diphenylmethyl und 1,3-Diphenylpropyl-2, wobei das Substitutionsmuster am Phenylkern jeweils o, m oder p sein kann;
- gegebenenfalls substituiertes Phenyl wie Phenyl, o-, m- oder p-Tolyl und durch Carbo-$C_1$-$C_{12}$-alkoxy substituiertes Phenyl wie o-, m- oder p-Carbomethoxyphenyl;
- ein Rest der Formel

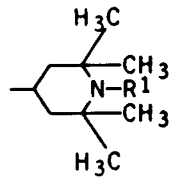

- Heterocyclen enthaltendes $C_1$-$C_{22}$-Alkyl wie

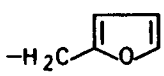 , 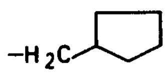 , 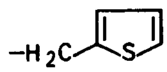 , 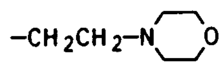 ,

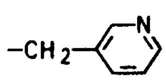 , 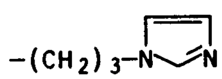 , 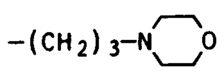 und 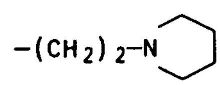

Bei Vorliegen einer durch $R^2$ bedingten Cyanguanidin-Struktur (a) kann $R^3$ zusätzlich für einen Rest der Formel

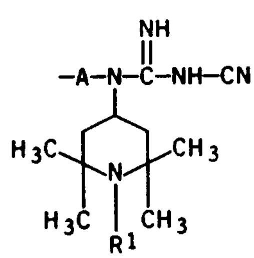

bei Vorliegen einer durch $R^2$ bedingten Biguanid-Struktur (b) zusätzlich für einen Rest der Formel

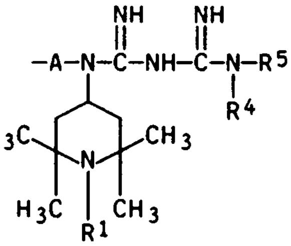

und bei Vorliegen einer durch $R^2$ bedingten Triazin-Struktur (c) zusätzlich für einen Rest der Formel

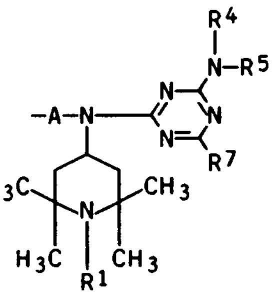

stehen, wobei in der letzten Formel $R^7$ dieselbe Bedeutung wie $R^6$ hat mit Ausnahme des Restes der Formel

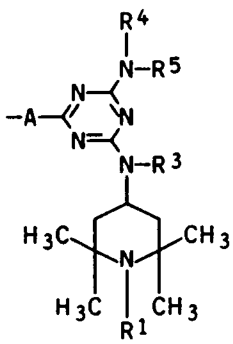

Hierbei kommen dem Brückenglied A folgende Bedeutungen zu:

- $C_2$- bis $C_{22}$-Alkylen und $C_5$- bis $C_{22}$-Cycloalkylen wie $-(CH_2)p-CH_2-$ (mit p = 1 bis 21),

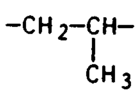 , 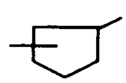 , 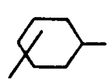 , 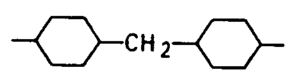 ,

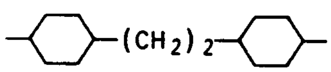 , 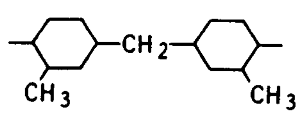 , 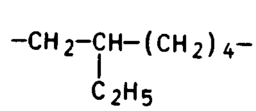 ,

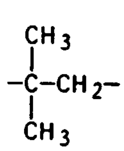 und 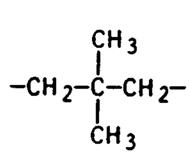 ;

- $C_8$-$C_{16}$-Phenylalkylen und Phenylen wie

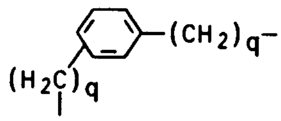 und 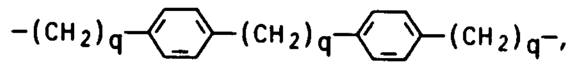

mit q = 0 - 4

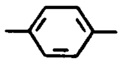 , 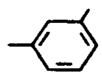 ;

- durch Ethersauerstoff-Atome, Stickstoff-Atome oder Heterocyclen unterbrochenes $C_4$-$C_{30}$-Alkylen wie $-(CH_2)_3O(CH_2)_4O(CH_2)_3-$, $-(CH_2)_3O(CH_2)_2O(CH_2)_2O(CH_2)_3-$,

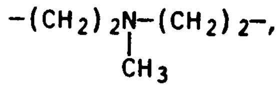

$-(C_3H_6O)_r-C_3H_6-$ mit r = 1 bis 9,

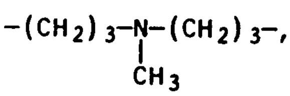 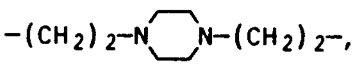 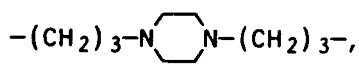

$-(CH_2)_3O(CH_2)_2O(CH_2)_3-$, 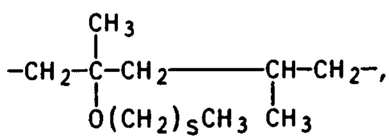

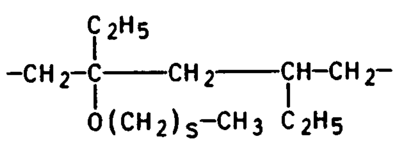 oder 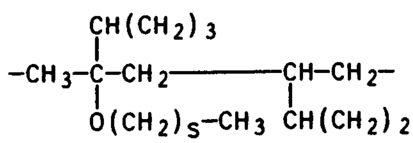

mit s = 0 bis 7.

Von den Resten $R^3$ werden Wasserstoff, geradkettiges und verzweigtes $C_1$- bis $C_8$-Alkyl, Cyclohexyl, Benzyl, Phenyl sowie die über das Brückenglied A verdoppelten Reste, d.h. bei Vorliegen einer durch $R^2$ bedingten Cyanguanidin-Struktur (a), ein Rest der Formel

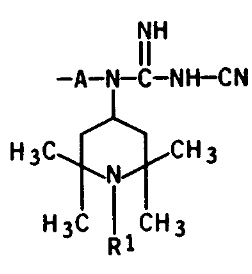

bei Vorliegen einer durch $R^2$ bedingten Biguanid-Struktur (b) ein Rest der Formel

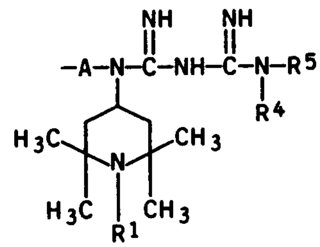

und bei Vorliegen einer durch $R^2$ bedingten Triazin-Struktur (c) ein Rest der Formel

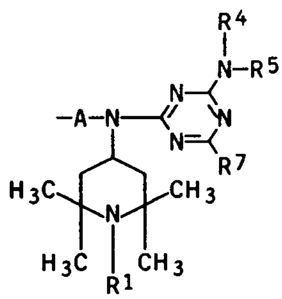

bevorzugt.

Das Brückenglied ist vorzugsweise $C_2$- bis $C_8$-Alkylen, insbesondere 1,2-Ethylen, 1,4-Butylen, 1,6-Hexylen oder 1,8-Octylen, weiterhin 1,4-Cyclohexyliden oder m- oder p-Phenylen.

Für $R^4$ und $R^5$ sind neben Wasserstoff z.B. zu nennen:

- geradkettiges und verzweigtes $C_1$-$C_{22}$-Alkyl, wie Methyl, Ethyl, n- und i-Propyl, n- und i-Butyl, n- und i-Pentyl, Hexyl, Octyl, Decyl, Dodecyl, Tetradecyl, Hexadecyl, Octadecyl, Pivalyl, 3,3-Dimethylbutyl-2, Neopentyl, 4-Methylpentyl-2 und 2-Ethylhexyl;
- $C_3$-$C_{22}$-Alkenyl wie Allyl, Butenyl, Pentenyl und Oleyl;
- $C_3$- bis $C_{12}$-Cycloalkyl wie Cyclopropyl, Cyclobutyl, Cyclopentyl, Cyclohexyl, Methylcyclohexyl, Cycloheptyl, Cyclooctyl, Cyclododecyl und Bicycloheptyl, von denen Cyclopentyl und vor allem Cyclohexyl bevorzugt werden;
- durch Ethersauerstoff- oder Stickstoff-Atome unterbrochenes $C_3$- bis $C_{22}$-Alkyl, welches zusätzlich Hydroxylgruppen tragen kann, wie $-(CH_2)_3N(CH_3)_2$, $-(CH_2)_3N(C_2H_5)_2$, $-(CH_2)_3-OCH_3$, $-(CH_2)_3-O-CH-(CH_3)_2$, $-(CH_2)_2-O-(CH_2)_2-OH$, $-CH_2-(CH_2)_2-CH_2-N(CH_3)_2$, $-(CH_2)_2-N[CH(CH_3)_2]_2$, $-(CH_2)_2-N(C_2H_5)_2$, $-(CH_2)_2-N(CH_3)_2$, $-(CH_2)_2-OCH_3$ und $-(CH_2)_2OCH_2CH_3$;
- gegebenenfalls substituiertes $C_7$- bis $C_{22}$-Phenyl- und Diphenylalkyl wie Benzyl, Methoxybenzyl, Methylbenzyl, Ethylbenzyl, Isopropylbenzyl, Trimethylbenzyl, Fluorbenzyl, Chlorbenzyl, Methylendioxybenzyl, $\beta$-Phenylethyl, $\gamma$-Phenylpropyl und $\delta$-Phenylbutyl, Dimethylaminobenzyl, Diphenylmethyl und 1,3-Diphenylpropyl-2, wobei das Substitutionsmuster am Phenylkern jeweils o, m oder p sein kann;
- Phenyl und substituiertes Phenyl wie 2-, 3- oder 4-Methylphenyl, 2-, 3- oder 4-Ethylphenyl, 2-, 3- oder 4-n-Propylphenyl, 2-, 3- oder 4-iso-Propylphenyl, 2-, 3- oder 4-n-Butylphenyl, 4-Hexylphenyl, 4-Octylphenyl, 4-Dodecylphenyl, 2,3-Dimethylphenyl, 2,6-Dimethylphenyl, 2,4-Dimethylphenyl, 2,4,6-Trimethylphenyl, 2,3,5,6-Tetramethylphenyl, 4-Cyclohexylphenyl, 2-, 3- oder 4-Methoxyphenyl, 2-, 3- oder 4-Ethoxyphenyl, 2-, 3- oder 4-Propoxyphenyl, 2-, 3- oder 4-Butoxyphenyl, 4-Hexoxyphenyl, 4-Octoxyphenyl, 4-Dodecoxyphenyl, 2-, 3- oder 4-Chlorphenyl, 2,3-Dichlorphenyl, 2,4-Dichlorphenyl, 2,6-Dichlorphenyl, 4-Hydroxyphenyl, 4-Phenylphenyl, 4-Benzylphenyl, 4-Cyanophenyl, 4-Methylaminophenyl, 4-Dimethylaminophenyl, 4-Ethylaminophenyl, 4-Diethylaminophenyl, 4-Propylaminophenyl, 4-Dipropylaminophenyl, 4-Butylaminophenyl, 4-Dibutylaminophenyl, 2-, 3- oder 4-Bromphenyl, 3,6-Diethoxyphenyl, 3,6-Dimethoxyphenyl, 2,4-Dimethoxyphenyl, 3-Chlor-6-methoxyphenyl, 2,4,5-Trimethylphenyl, 2,5-Dimethoxyphenyl, 2-Methoxy-5-methylphenyl, 3,4-Dimethoxyphenyl, 3,4-Diethoxyphenyl, 3,5-Dimethylphenyl;
- 5- oder 6-gliedrige heterocyclische aromatische Reste, wie z.B. 2-, 3- oder 4-Pyridyl, 2-(4,6-Dimethyl)-pyridyl, 2-(3-Hydroxy)-pyridyl, 2-(1,3,4)-Thiadiazolyl, 2-Thiazolyl, 3-(1,2,4)-Triazinyl, 3-Pyrazolyl, 2-Pyrazinyl oder 3-(5-Methyl)-isoxazolyl.

Dabei ist mindestens einer der Reste $R^4$ oder $R^5$ Phenyl, ein substituiertes Phenyl oder ein heterocyclischer aromatischer Rest.

In einer bevorzugten Ausführungsform steht einer der Reste $R^4$ oder $R^5$ für Wasserstoff, geradkettiges und verzweigtes $C_1$- bis $C_8$-Alkyl, Cyclohexyl und Benzyl und der andere für Phenyl, 4-$C_1$- bis $C_8$-Alkylphenyl, 4-$C_1$- bis $C_8$-Alkoxyphenyl, 4-Chlorphenyl, 4-Hydroxyphenyl und 4-Cyanophenyl.

Für $R^6$ ist neben Wasserstoff beispielsweise zu nennen:

- $C_1$- bis $C_{22}$-Alkyl wie Methyl, Ethyl, n- und i-Propyl, n- und i-Butyl, n- und i-Pentyl, Hexyl, Octyl, Decyl, Dodecyl, Tetradecyl, Hexadecyl, Octadecyl, Pivalyl, Neopentyl oder 2-Ethylhexyl;
- $C_2$- bis $C_{22}$-Alkenyl wie Vinyl, Allyl, Butenyl, Pentenyl oder Oleyl;
- $C_3$- bis $C_{12}$-Cycloalkyl wie Cyclopropyl, Cyclopentyl, Cyclohexyl, Methylcyclohexyl oder Cycloheptyl, von denen Cyclopentyl und vor allem Cyclohexyl bevorzugt werden;
- $C_7$- bis $C_{22}$-Phenyl- und Diphenylalkyl wie beispielsweise Benzyl, Methylbenzyl, $\beta$-Phenylethyl, $\gamma$-Phenylpropyl, $\delta$-Phenylbutyl, Carbethoxybenzyl, Carbomethoxybenzyl oder Ethoxybenzyl, wobei das Substitutionsmuster am Phenylkern jeweils o, m oder p sein kann;
- Phenyl und substituiertes Phenyl wie beispielsweise 2-, 3- oder 4-Methylphenyl, 2-,3- oder 4-Ethylphenyl, 2-, 3- oder 4-n-Propylphenyl, 2-, 3- oder 4-iso-Propylphenyl, 2-, 3- oder 4-n-Butylphenyl, 4-Hexylphenyl, 4-Octylphenyl, 4-Dodecylphenyl, 2,3-Dimethylphenyl, 2,6-Dimethylphenyl, 2,4-Dimethylphenyl, 2,4,6-Trimethylphenyl, 4-Cyclohexylphenyl, 2-, 3- oder 4-Methoxyphenyl, 2-, 3- oder 4-Ethoxyphenyl, 2-, 3- oder 4-Propoxyphenyl, 2-, 3- oder 4-Butoxyphenyl, 4-Hexoxyphenyl, 4-Octoxyphenyl, 4-Dodecoxyphenyl, 2-, 3- oder 4-Chlorphenyl, 2,3-Dichlorphenyl, 2,4-Dichlorphenyl, 2,6-Dichlorphenyl, 4-Hydroxyphenyl, 4-Phenylphenyl, 4-Benzylphenyl, 4-Cyanphenyl, 4-Methylaminophenyl, 4-Dimethylaminophenyl, 4-Ethylaminophenyl, 4-Diethylaminophenyl, 4-Propylaminophenyl, 4-Dipropylaminophenyl, 4-Butylaminophenyl, 4-Dibutylaminophenyl, 2-, 3- oder 4-Bromphenyl, 3,6-Diethoxyphenyl, 3,6-Dimethoxyphenyl, 2,4-Dimethoxyphenyl, 3-Chlor-6-methoxyphenyl, 2,4,5-Trimethylphenyl, 2,5-Dimethoxyphenyl, 2-Methoxy-5-methylphenyl, 3,4-Dimethoxyphenyl, 3,4-Diethoxyphenyl, 3,5-Dimethylphenyl, 3- oder 4-Carbomethoxyphenyl, 3- oder 4-Carboethoxyphenyl, 3- oder 4-Carbopropoxyphenyl, 3- oder 4-Carbobutoxyphenyl, 3- oder 4-Carbohexoxyphenyl, 3- oder 4-Carboheptoxyphenyl, 3- oder 4-Carbooctoxyphenyl oder 3- oder 4-Carbo-2'-ethylhexoxyphenyl;
- Pyridyl;
- durch Carbonyloxy unterbrochenes $C_2$- bis $C_{22}$-Alkyl wie beispielsweise

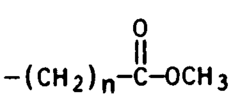 , 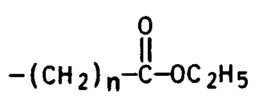 , 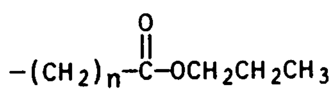 ,

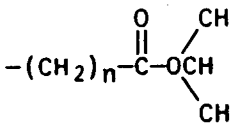 , 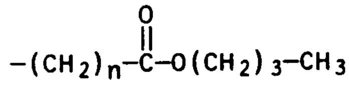 oder

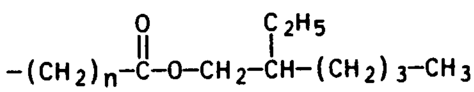 mit n = 1 bis 14 ;

- ein Rest der Formel

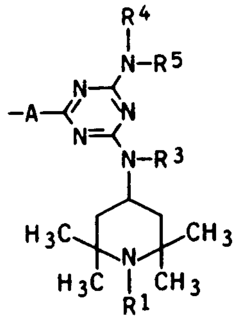

worin das Brückenglied A die oben angegebenen Bedeutungen hat.

Bevorzugte Reste $R^6$ sind Wasserstoff, geradkettiges und verzweigtes $C_1$- bis $C_8$-Alkyl, Phenyl, Benzyl, Cyclohexyl sowie die über das Brückenglied A verdoppelten Reste der Formel

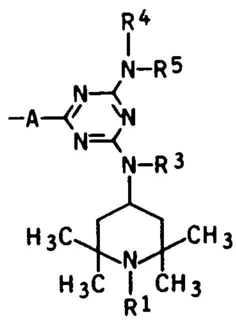

Die erfindungsgemäßen Polyalkylpiperidin-Derivate mit Cyanguanidin-Gruppierungen Ia lassen sich vorteilhafterweise durch an sich bekannte Umsetzungen von Aminen der allgemeinen Formel III oder IIIa

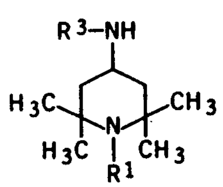

III

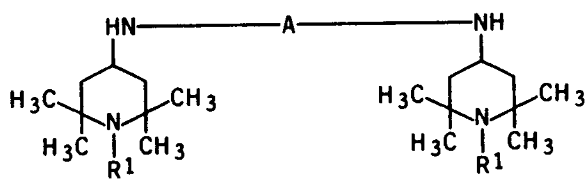

IIIa

mit Natriumdicyanamid herstellen. Derartige Umsetzungen sind z.B. in der Literaturstelle J. Chem. Soc. 1948, S. 1630-1636 (2) beschrieben. Die Amine III und IIIa sind z.B. aus der EP-A 316 582 (3) bekannt.

Die Umsetzung läßt sich in Wasser und in organischen Lösungsmitteln, vorzugsweise Alkoholen, insbesondere n-Butanol, oder in Gemischen hieraus durchführen. Man arbeitet hierbei üblicherweise in saurem Milieu, vorzugsweise in salzsaurem, bei Temperaturen von ca. 60 bis ca. 150 °C.

Je nach Substituenten und Lösungsmitteln können die Verbindungen Ia auch ganz oder teilweise in tautomeren Formen vorliegen, so gilt etwa das folgende Gleichgewicht:

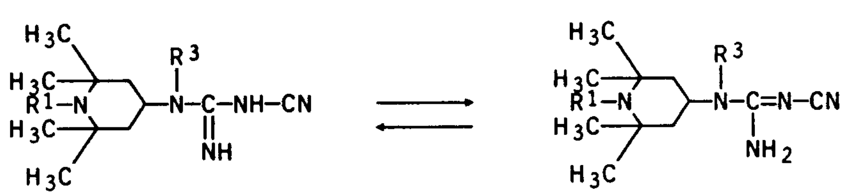

Ia

Analoge tautomere Formen können bei den Polyalkylpiperidin-Derivaten mit Biguanid-Gruppierungen Ib auftreten.

Die erfindungsgemäßen Biguanid-Derivate Ib lassen sich vorteilhafterweise durch an sich bekannte Umsetzungen aus den Cyanguanidinen Ia

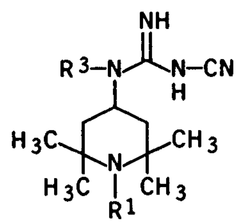

Ia

und Aminen der allgemeinen Formel $HNR^4R^5$ herstellen. Derartige Umsetzungen sind beispielsweise im Review-Artikel "The Chemistry of Biguanides", Fortschr. Chem. Forsch. 10, S. 375-472 (1968), (4) beschrieben.

Die Umsetzung läßt sich in Wasser und in organischen Lösungsmitteln, vorzugsweise Alkoholen, insbesondere Methanol, Ethanol, n-Propanol, iso-Propanol, n-Butanol und iso-Butanol, oder in Gemischen hieraus durchführen. Man arbeitet hierbei üblicherweise in saurem Milieu, vorzugsweise in salzsaurem, bei Temperaturen von ca. 60 bis ca. 150 ° C.

Die erfindungsgemäßen Polyalkylpiperidin-Derivate mit Triazin-Ringen Ic lassen sich vorteilhafterweise durch aus (4) an sich bekannte Umsetzungen aus den Biguaniden Ib

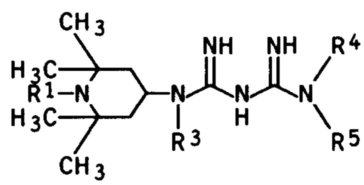

Ib

und Carbonsäuren oder Carbonsäure-Derivaten der allgemeinen Formeln $R^6$-COX, $R^6$-CN, XOC-A-COX, NC-A-COX oder NC-A-CN, wobei X für Hydroxyl, Chlor, Brom oder $C_1$- bis $C_4$-Alkoxy steht, herstellen.

Als Carbonsäuren oder Carbonsäure-Derivate können vor allem ein- oder zweibasische Carbonsäuren wie Ameisensäure, Essigsäure, Bernsteinsäure oder Adipinsäure, Mono- oder Biscarbonsäurehalogenide wie Acetylchlorid, Isobuttersäurechlorid, Benzoylchlorid oder Benzoylbromid, Mono- oder Biscarbonsäureester wie Essigsäuremethylester, Essigsäureethylester, Propionsäuremethylester, Buttersäuremethylester, Isobuttersäuremethylester, Benzoesäuremethylester, Bernsteinsäuredimethylester, Adipinsäuredimethylester, Korksäuredimethylester oder Sebacinsäuredimethylester sowie Mono-oder Biscarbonsäurenitrile wie Acetonitril oder Bernsteinsäuredinitril eingesetzt werden.

Die Umsetzung der Biguanide mit den Carbonsäuren bzw. Carbonsäure-Derivaten kann in Wasser, in einem organischen Lösungsmittel oder in Mischungen hieraus erfolgen. Organische Lösungsmittel können dabei Alkohole wie Methanol, Ethanol, n-Propanol, i-Propanol, n-Butanol, Ethylenglykol oder i-Butanol sein. Aber auch Ether wie z.B. Glykolmonobutylether, Glykoldimethylether, Glykoldibutylether kommen in Betracht. Ebenso können aromatische Kohlenwasserstoffe wie beispielsweise Benzol, Toluol, Xylol oder Mesitylen verwendet werden. Oft ist es von Vorteil, einen Reaktanden als Lösungsmittel einzusetzen, wenn dieser flüssig ist, also beispielsweise bei Carbonsäureestern wie Essigsäureethylester, Propionsäuremethylester oder Adipinsäuredimethylester oder bei Carbonsäuren wie Ameisensäure.

Zweckmäßigerweise benutzt man für die Umsetzung eine Hilfsbase wie beispielsweise Natriummethylat, Natriumethylat, Triethylamin, Tributylamin, Natronlauge, Kalilauge, Natriumcarbonat oder Kaliumcarbonat. Man führt die Umsetzung in der Regel bei Temperaturen von ca. 60 bis ca. 150 ° C durch.

Besonders vorteilhaft ist ein mehrstufiges Herstellungsverfahren für die Triazine Ic

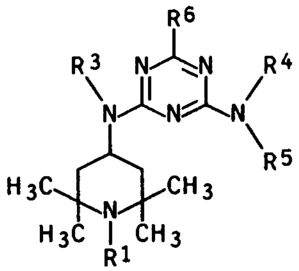

Ic

ausgehend von den Aminen III bzw. IIIa unter Verwendung der oben beschriebenen Reaktionsschritte.

Die erfindungsgemäßen Polyalkylpiperidin-Derivate mit Cyanguanidin-Gruppierungen Ia sowie die erfindungsgemäßen Polyalkylpiperidin-Derivate und Biguanid-Gruppierungen Ib stellen wertvolle Zwischenprodukte für die Herstellung von hauptsächlich als Stabilisatoren und Lichtschutzmittel für organisches Material verwendeten Triazin-Derivaten Ic sowie für weitere potentielle Stabilisatoren und Lichtschutzmittel dar.

Die erfindungsgemäßen Verbindungen I können in Form der freien Basen oder als Säureadditionssalze vorliegen. Geeignete Anionen stammen z.B. von anorganischen Säuren und insbesondere von organischen Carbonsäuren sowie organischen Sulfonsäuren.

Als anorganische Anionen sind z.B. Chlorid, Bromid, Sulfat, Dicyanamid, Methosulfat, Tetrafluoroborat, Phosphat und Rhodanid zu nennen.

Als Carbonsäureanionen kommen z.B. Formiat, Acetat, Propionat, Hexanoat, Cyclohexancarboxylat, Lactat, Stearat, Dodecylbenzoat, Acrylat, Methacrylat, Citrat, Malonat oder Succinat sowie Anionen von Polycarbonsäuren mit bis zu 3000 COOH-Gruppen in Betracht.

Sulfonsäure-Anionen sind beispielsweise Benzolsulfonat oder Tosylat.

Die erfindungsgemäßen Polyalkylpiperidin-Derivate mit Cyanguanidin-Gruppierungen Ia und mit Triazin-Ringen Ic eignen sich in hervorragender Weise zum Stabilisieren von organischem Material gegen die Einwirkung von Licht, Sauerstoff und Wärme. Sie sind auch wirksam als Metalldesaktivator. Sie werden dem zu stabilisierenden organischen Material in einer Konzentration von 0,01 bis 5 Gew.-%, vorzugsweise von 0,02 bis 1 Gew.-%, bezogen auf das organische Material, vor, während oder nach seiner Herstellung zugesetzt.

Unter organischem Material sind beispielsweise kosmetische Präparate wie Salben und Lotionen, Arzneimittelformulierungen wie Pillen und Zäpfchen oder Vorprodukte für Kunststoffe und Lacke, insbesondere jedoch Kunststoff und Lacke selbst, zu verstehen.

Gegenstand der vorliegenden Erfindung ist außerdem gegen die Einwirkung von Licht, Sauerstoff und Wärme stabilisiertes organisches Material, insbesondere Kunststoff und Lacke, welches die Verbindungen Ia oder Ic in den oben angegebenen Konzentrationen enthält.

Zur Vermischung der erfindungsgemäßen Verbindungen Ia bzw. Ic vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Verbindungen Ia bzw. Ic stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-$\beta$-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, Tris-(3,5-di-tert.-butyl-4-hydroxybenzyl)-isocyanurat, Tris-[$\beta$-(3,5-di-tert.-butyl-4-hydroxyphenyl)-propionylethyl]isocyanurat, Tris-(2,6-dimethyl-3-hydroxy-4-tert.-butylbenzyl)-isocyanurat und Pentaerythrit-tetrakis-[$\beta$-(3,5-di-tert.-butyl-4-hydroxyphenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-($\beta$-laurylthiopropionat) und Pentaerythrittetrakis-

($\beta$-hexylthiopropionat) genannt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, $\alpha$-Cyanozimtsäurederivate, Benzimidazolcarbonsäureanilide, Nickelverbindungen oder Oxalsäuredianilide.

Als Kunststoffe, die durch die erfindungsgemäßen Verbindungen Ia bzw. Ic stabilisert werden können, seien beispielsweise genannt:

Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder $\alpha$-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);

halogenhaltige Polymere, wie z.B. Polyvinylchlorid, Polyvinylfluorid, Polyvinylidenfluorid sowie deren Copolymere;

Polymere, die sich von $\alpha$,$\beta$-ungesättigten Säuren und deren Derivaten ableiten, wie Polyacrylate, Polymethacrylate, Polyacrylamide und Polyacrylnitrile;

Polymere, die sich von ungesättigten Alkoholen und Aminen bzw. von deren Acrylderivaten oder Acetalen ableiten, z.B. Polyvinylalkohol und Polyvinylacetat;

Polyurethane, Polyamide, Polyharnstoffe, Polyester, Polycarbonate, Polysulfone, Polyethersulfone und Polyetherketone.

Weiterhin können mit den erfindungsgemäßen Verbindungen Ia bzw. Ic Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen Ia bzw. Ic können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen Ia bzw. Ic zum Stabilisieren von Polyamiden sowie ABS- und SAN-Polymeren, insbesondere von Formmassen hieraus, verwendet.

Ein weiteres bevorzugtes Einsatzgebiet ist die Stabilisierung von Polypropylen und Polyamid, insbesondere von Fasern hieraus.

Die erfindungsgemäßen Verbindungen Ia und Ic zeichnen sich durch eine gute Verträglichkeit mit den üblichen Kunststoffarten und durch eine gute Löslichkeit in den üblichen Lacksystemen aus. Sie haben in der Regel keine oder nur eine sehr geringe Eigenfarbe, sind bei den üblichen Kunststoff- und Lack-Verarbeitungstemperaturen stabil und nicht flüchtig und bewirken vor allen Dingen eine lange Schutzdauer der mit ihnen behandelten Materialien.

Herstellungsbeispiele

Beispiel 1a

53,4 g 2,2,6,6-Tetramethyl-4-aminopiperidin, 53,4 g Natriumdicyanamid und 54 g konzentrierte Salzsäure wurden in 150 ml Wasser 60 h zum Rückfluß erhitzt. Nach Absaugen, Waschen mit Wasser und Methanol sowie Trocknen im Vakuum erhielt man 80,1 g der Verbindung der Formel

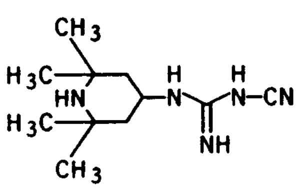

als farblosen Feststoff vom Schmp. 266-267 ° C.

| | | | |
|---|---|---|---|
| Ber.: | C 59,2 | H 9,5 | N 31,4 |
| Gef.: | C 58,7 | H 9,4 | N 31,4 |

Beispiel 1b

42,1 g 2,2,6,6-Tetramethyl-4-aminopiperidin, 26,7 g Natriumdicyanamid und 27 g konzentrierte Salzsäure wurden in 200 ml n-Butanol 20 h zum Rückfluß erhitzt. Nach dem Abkühlen wurde der ausgefallene Niederschlag abgesaugt, mit 200 ml Ethanol gewaschen, mit 300 ml Wasser ausgekocht und getrocknet. Man erhielt 45,6 g der Verbindung aus Beispiel 1a vom Schmp. 266-267 ° C.

Beispiel 2

116,8 g 4-n-Butylamino-2,2,6,6-tetramethylpiperidin, 53,4 g Natriumdicyanamid und 55 g konzentrierte Salzsäure wurden in 150 ml Wasser 8 h zum Sieden erhitzt. Nach Abkühlen und Stehenlassen schied sich ein semikristalliner Niederschlag ab. Nach dem Abdekantieren des Wassers wurde der Rückstand in 150 ml heißem Ethanol aufgenommen, die Lösung wurde filtriert und nach dem Abkühlen wurde der ausgefallene Niederschlag abgesaugt. Man erhielt 69,1 g der Verbindung der Formel

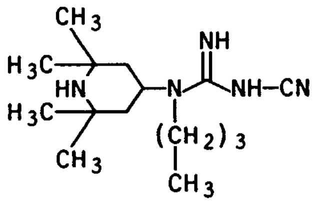

als farblosen Feststoff vom Schmp. 188-189 ° C.

| | | | |
|---|---|---|---|
| Ber.: | C 64,5 | H 10,5 | N 25,1 |
| Gef.: | C 64,2 | H 10,6 | N 25,0 |

Beispiel 3

60,0 g 4-n-Hexylamino-2,2,6,6-tetramethylpiperidin, 39,7 g Natriumdicyanamid und 25 g konzentrierte Salzsäure wurden in 150 ml Wasser 15 h zum Rückfluß erhitzt. Nach Abkühlen und Zugabe von 10 ml 50 %iger Natronlauge wurde der ausgefallene Niederschlag abgesaugt, mit Wasser gewaschen und aus Acetonitril umkristallisiert. Man erhielt 62 g der Verbindung der Formel

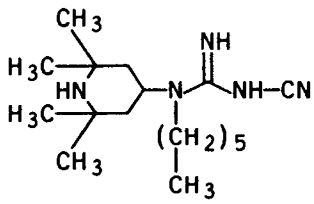

als farblosen Feststoff vom Schmp. 193-194 ° C.

| | | | |
|---|---|---|---|
| Ber.: | C 66,4 | H 10,8 | N 22,8 |
| Gef.: | C 66,4 | H 10,9 | N 22,9 |

Beispiel 4

67 g 4-n-Octylamino-2,2,6,6-tetramethylpiperidin, 39,7 g Natriumdicyanamid und 25 g konzentrierte Salzsäure wurden in 150 ml Wasser 13 h zum Sieden erhitzt und wie in Beispiel 3 aufgearbeitet. Man erhielt 64 g der Verbindung der Formel

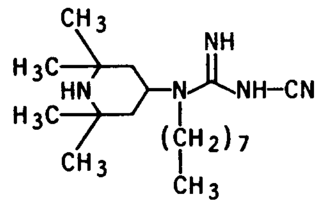

als farblosen Feststoff vom Schmp. 173-175 ° C.

| | | | |
|---|---|---|---|
| Ber.: | C 68,0 | H 11,1 | N 20,9 |
| Gef.: | C 68,0 | H 11,2 | N 21,1 |

Beispiel 5

238 g 4-Cyclohexylamino-2,2,6,6-tetramethylpiperidin, 98 g Natriumdicyanamid und 100 g konzentrierte Salzsäure wurden 10,5 h zum Sieden erhitzt. Nach Zugabe von 98 g Natriumdicyanamid und 50 ml n-Butanol wurde die Reaktionsmischung weitere 14 h zum Sieden erhitzt. Der ausgefallene Niederschlag wurde nach dem Abkühlen in Natronlauge verrührt, abgesaugt und aus Acetonitril umkristallisiert. Man erhielt 140 g der Verbindung der Formel

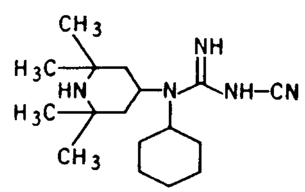

als farblosen Feststoff vom Schmp. 230-233 ° C.

| | | | |
|---|---|---|---|
| Ber.: | C 66,8 | H 10,2 | N 22,9 |
| Gef.: | C 66,7 | H 10,3 | N 23,3 |

Beispiel 6

78,8 g

N,N' -Bis(2',2',6',6'-tetramethyl-4' -piperidinyl)-1,6-hexamethylendiamin,

35,6 g Natriumdicyanamid und 40 g konzentrierte Salzsäure wurden in 130 ml Wasser 4,5 h zum Sieden erhitzt. Nach Zugabe von 17 g Natriumdicyanamid und 20 g konzentrierter Salzsäure wurde die Reaktionsmischung weitere 8,5 h zum Sieden erhitzt. Nach Abkühlen und Absaugen wurde der ausgefallene Niederschlag in Natronlauge verrührt, abgesaugt, mit Wasser neutral gewaschen und aus Dimethylformamid umkristallisiert. Man erhielt 37 g der Verbindung der Formel

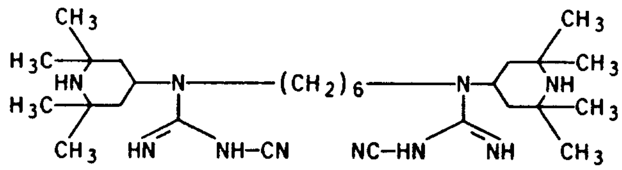

als farblosen Feststoff vom Schmp.: 305-306 ° C.

| | | | |
|---|---|---|---|
| Ber.: | C 63,6 | H 9,9 | N 26,5 |
| Gef.: | C 63,6 | H 9,9 | N 26,4 |

Beispiel 7

33,5 g 2,2,6,6-Tetramethyl-4-piperidinyl-cyanguanidin aus Beispiel 1, 18,4 g p-Anisidin und 25 ml konzentrierte Salzsäure wurden in 100 ml Wasser 5 h zum Sieden erhitzt. Die Lösung wurde filtriert und das Filtrat mit Natronlauge alkalisch gestellt. Der ausgefallene Niederschlag wurde abgesaugt, getrocknet und aus Cyclohexan umkristallisiert. Man erhielt 30 g des Produktes der Formel

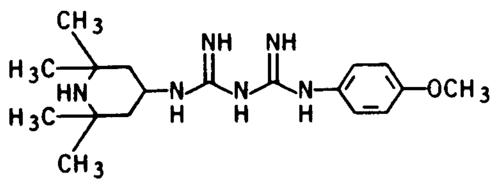

als farblosen Feststoff vom Schmp. 132-136 ° C.

Beispiel 8

44,6 g 2,2,6,6-Tetramethyl-4-piperidinyl-cyanguanidin aus Beispiel 1, 26,5 g p-Chloranilin und 40 g konzentrierte Salzsäure wurden in 100 ml Wasser 5 h zum Sieden erhitzt. Nach dem Abkühlen wurde die Lösung mit Natronlauge alkalisch gestellt. Die wäßrige Phase wurde vom ausgefallenen schmierigen Niederschlag abdekantiert und der Rückstand wurde in einer Mischung aus 150 ml Acetonitril und 450 ml Wasser gerührt. Der nun ausgefallene feinkristalline Niederschlag wurde abgesaugt, getrocknet und mit Methylcyclohexan ausgekocht. Man erhielt 50 g der Verbindung der Formel

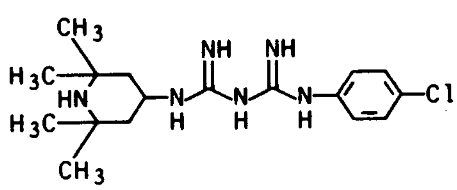

als farblosen Feststoff vom Schmp. 152-155 ° C.

Beispiel 9

44,6 g 2,2,6,6-Tetramethyl-4-piperidinyl-cyanguanidin aus Beispiel 1, 18,6 g Anilin und 40 g konzentrierte Salzsäure wurden in 100 ml Wasser 3 h zum Sieden erhitzt. Nach Zugabe von 100 g Eis wurde mit Natronlauge alkalisch gestellt, 150 ml Wasser zugegeben und 30 Minuten gerührt. Der ausgefallene Niederschlag wurde abgesaugt, mit Wasser gewaschen, getrocknet und mit Cyclohexan ausgekocht. Man erhielt 29 g der Verbindung der Formel

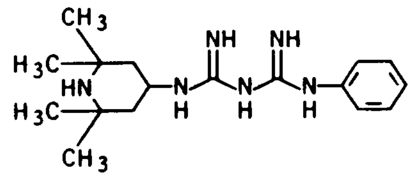

als farblosen Feststoff vom Schmp. 153-156 °C.

Beispiel 10

44,6 g 2,2,6,6-Tetramethyl-4-piperidinyl-cyanguanidin aus Beispiel 1, 21,4 g p-Toluidin und 40 g konzentrierte Salzsäure wurden wie in Beispiel 9 umgesetzt und aufgearbeitet. Nach Umkristallisation aus Toluol erhielt man 29 g der Verbindung der Formel

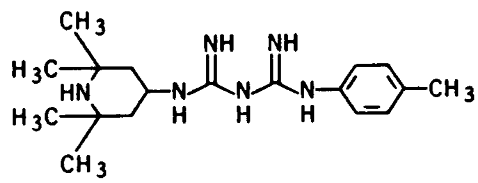

als farblosen Feststoff vom Schmp. 156-158 °C.

Beispiel 11

100,3 g 2,2,6,6-Tetramethyl-4-piperidinyl-cyanguanidin aus Beispiel 1, 61,6 g p-Phenetidin und 90 g konzentrierte Salzsäure wurden wie in Beispiel 9 umgesetzt und aufgearbeitet. Nach Umkristallisation aus Methylcyclohexan erhielt man 52 g der Verbindung der Formel

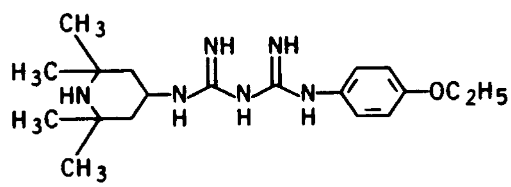

als farblosen Feststoff vom Schmp. 130-131 °C.

Beispiel 12

50 g N-Octyl-N-(2,2,6,6-tetramethyl-4-piperidinyl)-cyanguanidin aus Beispiel 4, 20,4 g p-Phenetidin und 29,8 g konzentrierte Salzsäure wurde in 150 ml Wasser 6 h zum Sieden erhitzt. Die Lösung wurde mit Natronlauge alkalisch gestellt und mit Dichlormethan ausgeschüttelt. Nach der Phasentrennung wurde das organische Lösungsmittel im Wasserstrahlvakuum entfernt, man erhielt als Rückstand die Verbindung der Formel

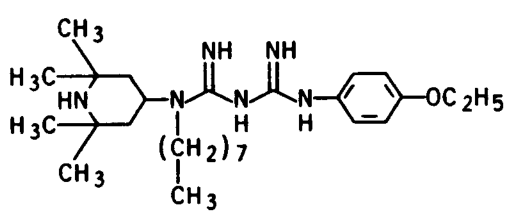

als hochviskoses Öl.

| Ber.: | C 68,6 | H 10,2 | N 17,8 |
|---|---|---|---|
| Gef.: | C 68,5 | H 10,5 | N 17,8 |

Beispiel 13

48 g N-Hexyl-N-(2,2,6,6-tetramethyl-4-piperidinyl)-cyanguanidin aus Beispiel 3, 21,4 g p-Phenetidin und 31,2 g konzentrierte Salzsäure wurden in 150 ml Wasser 5,5 h zum Sieden erhitzt. Unter Kühlung wurde mit Natronlauge alkalisch gestellt, die wäßrige Phase abdekantiert und der verbliebene Rückstand in Toluol gelöst. Die Toluolphase wurde mit Wasser gewaschen, nach der Phasentrennung wurde das Toluol im Wasserstrahlvakuum entfernt. Es verblieben 50 g eines zähen Rückstandes, der die Struktur der Formel

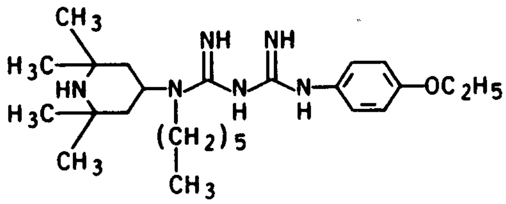

besaß.

Beispiel 14

24 g

N,N'-Bis(cyanamidino)-N,N'-bis(2',2',6',6'-tetramethyl-4'-piperidinyl)-1,6-hexamethylendiamin

aus Beispiel 6, 12,35 g p-Phenetidin und 18 g konzentrierte Salzsäure wurden in 125 ml Wasser 8,5 h zum Sieden erhitzt. Unter Eiskühlung wurde mit Natronlauge alkalisch gestellt, der ausgefallene Niederschlag wurde abgesaugt, getrocknet und unter Zusatz von Aktivkohle aus Methylcyclohexan umkristallisiert. Man erhielt 22,6 g der Verbindung der Formel

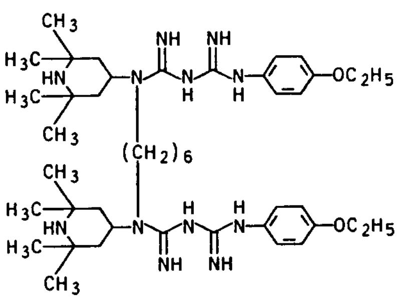

als farblosen Feststoff vom Schmp. 95-97 ° C.

Beispiel 15

50 g N-Cyclohexyl-N-(2,2,6,6-tetramethyl-4-piperidinyl)-cyanguanidin aus Beispiel 5, 22,5 g p-Phenetidin und 33 g konzentrierte Salzsäure wurden in 150 ml Wasser 5,5 h zum Sieden erhitzt. Unter Kühlung wurde mit Natronlauge alkalisch gestellt und mit Dichlormethan ausgeschüttelt. Die organische Phase wurde im Wasserstrahlvakuum eingeengt, der Rückstand aus Acetonitril umkristallisiert. Man erhielt 58 g der Verbindung der Formel

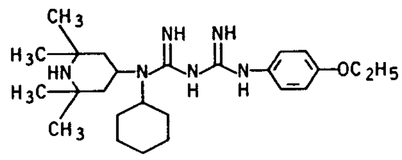

als farblosen Feststoff vom Schmp. 115-116 ° C.

Beispiel 16

66,9 g 2,2,6,6-Tetramethyl-4-piperidinyl-cyanguanidin aus Beispiel 1, 32,1 g N-Methylanilin und 60 g konzentrierte Salzsäure wurden in 150 ml Wasser 7,5 h zum Sieden erhitzt. Unter Kühlung wurde mit Natronlauge stark alkalisch gestellt, die wäßrige Phase wurde abdekantiert und der Rückstand in Essigsäureethylester aufgenommen. Nach Waschen mit Wasser wurde die organische Phase im Wasserstrahlvakuum vom Lösungsmittel befreit und der Rückstand aus Acetonitril umkristallisiert. Man erhielt 43 g der Verbindung der Formel

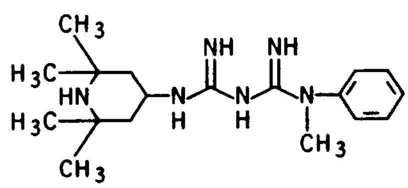

als farblosen Feststoff vom Schmp. 135-137 ° C.

Beispiel 17

66,9 g 2,2,6,6-Tetramethyl-4-piperidinyl-cyanguanidin aus Beispiel 1, 44,7 g N-Butylanilin und 60 g konzentrierte Salzsäure wurden in 150 ml Wasser 7,5 h zum Sieden erhitzt. Mit Natronlauge wurde stark alkalisch gestellt. Nach Zugabe von Dichlormethan fiel ein Niederschlag aus, der abfiltriert wurde. Die Methylenchloridphase wurde eingeengt, wobei weiterer Niederschlag ausfiel, die beiden Niederschläge wurden vereinigt und aus Cyclohexan unter Zusatz von Aktivkohle umkristallisiert. Man erhielt 62 g des Produktes der Formel

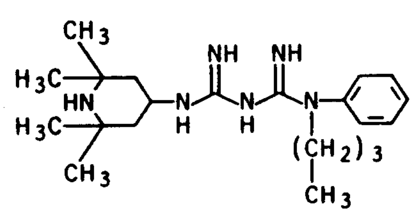

als farblosen Feststoff vom Schmp. 65-68 ° C.

Beispiel 18

35 g

1-(2',2',6',6'-Tetramethyl-4'-piperidinyl)-5-(p-chlorphenyl)-biguanid

aus Beispiel 8, 18 g 30 gew.-%ige methanolische Natriummethylatlösung und 30,2 g Isobuttersäuremethylester wurden in 100 ml Toluol 5 h erhitzt und die Niedersieder dabei abdestilliert. Nach Erreichen einer Siedetemperatur von 110 ° C wurden 100 ml Wasser zugesetzt und die Phasen in der Hitze getrennt. Aus der Toluolphase fiel beim Abkühlen ein Niederschlag aus, der abgesaugt und mit wenig Acetonitril

gewaschen wurde. Nach dem Trocknen erhielt man 29,2 g der Verbindung der Formel

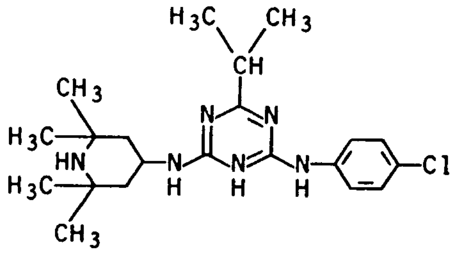

als farblosen Feststoff vom Schmp. 190-192 ° C.

| | | | | |
|---|---|---|---|---|
| Ber.: | C 62,6 | H 7,7 | N 20,8 | Cl 8,8 |
| Gef.: | C 62,9 | H 7,8 | N 20,7 | Cl 8,6 |

Beispiel 19

Zu 26 g

1-(2',2',6',6'-Tetramethyl-4'-piperidinyl)-5-(p-methoxyphenyl)-bi- guanid,

hergestellt durch Umsetzung von 2,2,6,6-Tetramethyl-4-piperidinylcyanguanidin aus Beispiel 1 mit p-Anisidin analog zu Beispiel 9, und 19 g 30 gew.-%iger methanolischer Natriummethylatlösung in 100 ml Toluol wurden bei 110 ° C Innentemperatur unter Abdestillieren der Tiefsieder innerhalb von 9 h 100 ml Essigester so zugesetzt, daß die Innentemperatur bei 105 ° C lag. Man arbeitete wie in Beispiel 18 auf, behandelte die Toluolphase mit Aktivkohle und Magnesiumsulfat und destillierte anschließend das Toluol im Wasserstrahlvakuum ab. Nach Umkristallisation des Rückstandes aus Acetonitril erhielt man 11,7 g der Verbindung der Formel

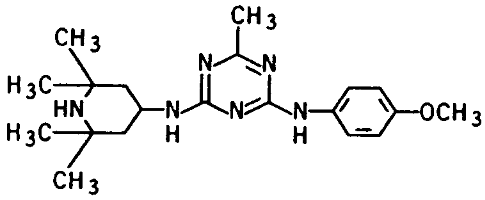

als farblosen Feststoff vom Schmp. 175-177 ° C.

| | | | |
|---|---|---|---|
| Ber.: | C 64,8 | H 8,2 | N 22,7 |
| Gef.: | C 64,9 | H 8,4 | N 22,6 |

Beispiel 20

24,8 g

1-(2',2',6',6'-Tetramethyl-4'-piperidinyl)-5-(p-methylphenyl)-bi- guanid

aus Beispiel 10 und 13,5 g einer 30 gew.-%igen methanolischen Natriummethylatlösung wurden in 75 ml Essigsäureethylester 4 h zum Rückfluß erhitzt. Nach Abkühlen wurden 150 ml Wasser zugegeben, die

Phasen getrennt und die organische Phase wurde über Magnesiumsulfat getrocknet. Nach Filtration und Abdestillieren des Lösungsmittels im Wasserstrahlvakuum wurde der Rückstand aus Acetonitril umkristallisiert. Man erhielt 12,1 g der Verbindung der Formel

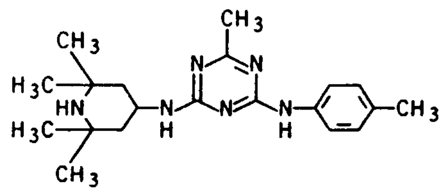

als farblosen Feststoff vom Schmp. 182-184 ° C.

| | | | |
|---|---|---|---|
| Ber.: | C 67,8 | H 8,5 | N 23,7 |
| Gef.: | C 67,8 | H 8,6 | N 23,7 |

Beispiel 21

16,5 g

1-(2',2',6',6'-Tetramethyl-4'-piperidinyl)-5-(p-methylphenyl)-bi- guanid

aus Beispiel 10 und 9 g einer 30 gew.-%igen methanolischen Natriummethylatlösung wurden in 190 ml Benzoesäuremethylester 4 h auf 125 ° C erhitzt. Nach dem Abkühlen wurden 250 ml Petrolether zugesetzt, es wurde vom Unlöslichen abfiltriert und das Filtrat wurde eingeengt. Der Rückstand wurde zweimal mit Acetonitril ausgekocht. Man erhielt 9,2 g der Verbindung der Formel

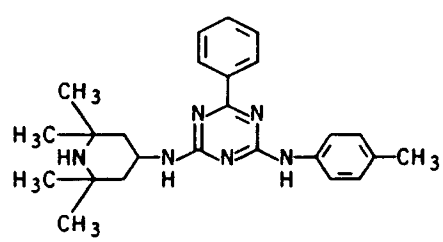

als farblosen Feststoff vom Schmp. 178-180 ° C.

| | | | |
|---|---|---|---|
| Ber.: | C 72,1 | H 7,7 | N 20,2 |
| Gef.: | C 72,1 | H 7,9 | N 20,2 |

Beispiel 22

40 g

1-Butyl-1-(2',2',6',6'-tetramethyl-4'-piperidinyl)-5-(p-methyl- phenyl)-biguanid,

hergestellt durch Umsetzung von N-Hexyl-N-(2,2,6,6-tetramethyl-4-piperidinyl)-cyanguanidin aus Beispiel 3 mit p-Toluidin analog zu Beispiel 9, 19 g einer 30 gew.-%igen methanolischen Natriummethylatlösung und 10,8 g Benzoesäuremethylester wurden analog zu Beispiel 18 in 150 ml Toluol 9 h erhitzt. Nach Abkühlen auf Raumtemperatur wurde filtriert und das Filtrat im Wasserstrahlvakuum eingeengt. Der Rückstand wurde zweimal aus Acetonitril umkristallisiert, man erhielt 7,2 g der Verbindung der Formel

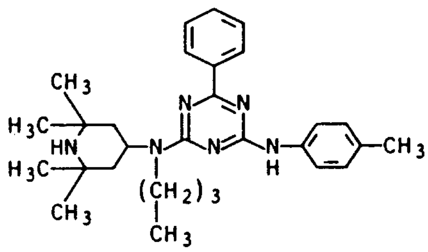

als farblosen Feststoff vom Schmp. 159-161 ° C.

| | | | |
|---|---|---|---|
| Ber.: | C 73,7 | H 8,5 | N 17,8 |
| Gef.: | C 73,1 | H 8,7 | N 17,7 |

Beispiel 23

25 g

1-Hexyl-1-(2',2',6',6'-tetramethyl-4'-piperidinyl)-5-(p-ethoxy- phenyl)-biguanid

aus Beispiel 13 wurden in 50 ml Ameisensäure 20 h zum Sieden erhitzt. Nach dem Abkühlen wurde mit Eiswasser verdünnt, mit Natronlauge stark alkalisch gestellt und mit Dichlormethan extrahiert. Die organische Phase wurde im Wasserstrahlvakuum eingeengt und der Rückstand aus n-Hexan umkristallisiert. Man erhielt 17,5 g der Verbindung der Formel

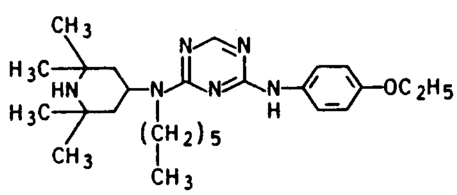

als farblosen Feststoff vom Schmp. 130-132 ° C.

| | | | |
|---|---|---|---|
| Ber.: | C 68,7 | H 9,3 | N 18,5 |
| Gef.: | C 68,2 | H 9,3 | N 18,2 |

Beispiel 24

30 g

1-Octyl-1-(2',2',6',6'-tetramethyl-4'-piperidinyl)-5-(p-ethoxy- phenyl)-biguanid

aus Beispiel 12 wurden in 75 g Ameisensäure analog Beispiel 23 umgesetzt und aufgearbeitet. Nach Umkristallisation aus Acetonitril erhielt man 20,5 g der Verbindung der Formel

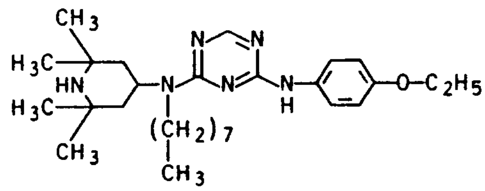

als farblosen Feststoff vom Schmp. 122-130 ° C.

| | | | |
|---|---|---|---|
| Ber.: | C 69,7 | H 9,6 | N 17,4 |
| Gef.: | C 69,8 | H 9,8 | N 17,4 |

Beispiel 25

25 g

1-Hexyl-1-(2',2',6',6'-tetramethyl-4'-piperidinyl)-5-(p-ethoxy-phenyl)-biguanid

aus Beispiel 13, 10,1 g einer 30 gew.-%igen methanolischen Natriummethylatlösung und 3,8 g Adipinsäuredimethylester wurden in 50 ml Ethylenglykolmonobutylether 35 h auf 100 ° C erhitzt. Nach dem Abkühlen wurden 25 ml konzentrierte Salzsäure und nach Stehenlassen über Nacht 200 ml Wasser zugegeben. Nach Zugabe von Natronlauge wurde die alkoholische Mischung mit Dichlormethan extrahiert. Die organische Phase wurde im Wasserstrahlvakuum eingeengt und der Rückstand aus Essigsäureethylester umkristallisiert. Man erhielt 4,6 g der Verbindung der Formel

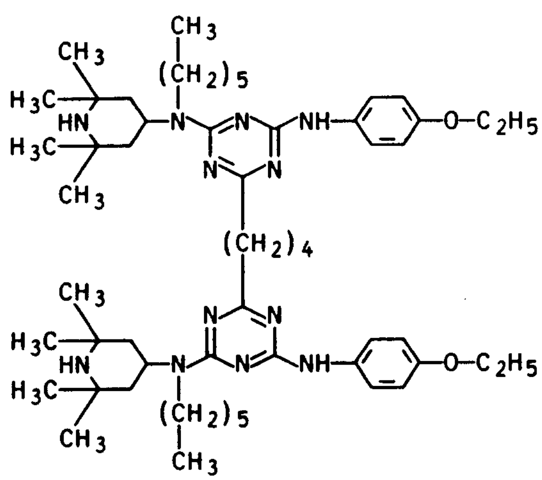

als farblosen Feststoff vom Schmp. 190-191 ° C.

| | | | |
|---|---|---|---|
| Ber.: | c 69,8 | H 9,4 | N 17,4 |
| Gef.: | C 69,4 | H 9,5 | N 17,4 |

**Patentansprüche**

**1.** Polyalkylpiperidin-Derivate der allgemeinen Formel I

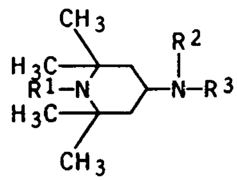

I

in der

$R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,

$R^2$ für einen Rest der Formel (a)

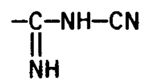

(a)

für einen Rest der Formel (b)

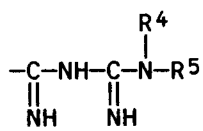

(b)

oder für einen Rest der Formel (c)

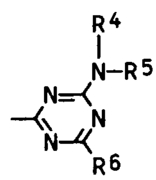

(c)

steht, wobei

$R^4$ und $R^5$ Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl, durch Ethersauerstoff- oder Stickstoff-Atome unterbrochenes $C_3$- bis $C_{22}$-Alkyl, welches zusätzlich Hydroxylgruppen tragen kann, Phenyl, das unsubstituiert oder ein- bis vierfach durch $C_1$- bis $C_{12}$-Alkyl, $C_3$- bis $C_{12}$-Cycloalkyl, $C_1$- bis $C_{12}$-Alkoxy, Chlor, Brom, $C_1$- bis $C_{12}$-Alkylamino, $C_1$- bis $C_{12}$-Dialkylamino, Phenyl, Benzyl, Hydroxyl oder Cyano substituiert sein kann, oder einen 5- oder 6-gliedrigen aromatischen Heterocyclus bedeuten, mit der Maßgabe, daß mindestens einer der Reste $R^4$ oder $R^5$ ein aromatischer Rest ist, und

$R^6$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-

Alkyl oder einen Rest der Formel

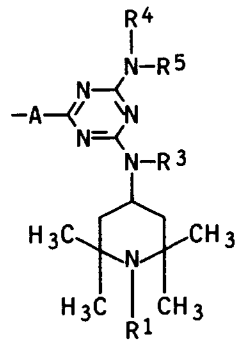

worin

A $C_2$- bis $C_{22}$-Alkylen, $C_8$- bis $C_{22}$-Cycloalkylen, $C_8$- bis $C_{16}$-Phenylalkylken, Phenylen oder durch Ethersauerstoff-Atome, Stickstoff-Atome oder 5- oder 6-gliedrige Heterocyclen unterbrochenes $C_4$- bis $C_{30}$-Alkylen bedeutet,

bezeichnet, und

$R^3$ Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl oder Bicycloalkyl, durch Ethersauerstoff- oder Stickstoff-Atome unterbrochenes $C_4$- bis $C_{22}$-Alkyl, welches zusätzlich Hydroxylgruppen tragen kann, Phenyl, welches durch ein bis drei Methyl- oder Carbo-$C_1$-$C_{12}$-alkoxy-Gruppen substituiert sein kann, heterocyclische Reste enthaltendes $C_1$-bis $C_{22}$-Alkyl, einen Rest der Formel

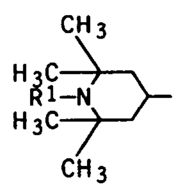

oder bei Vorliegen des Restes (a) für $R^2$ einen Rest der Formel

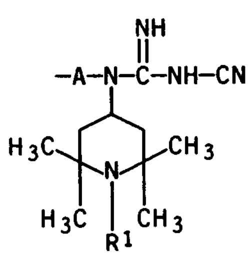

oder bei Vorliegen des Restes (b) für $R^2$ einen Rest der Formel

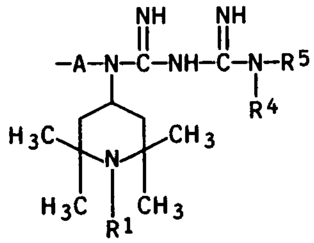

oder bei Vorliegen des Restes (c) für $R^2$ einen Rest der Formel

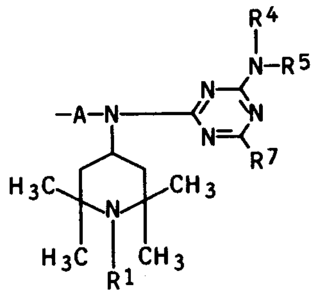

wobei $R^7$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl bedeutet,

bezeichnet,

sowie Tautomere und Säureadditionssalze der Verbindungen I.

**2.** Polyalkylpiperidin-Derivate I nach Anspruch 1, bei denen der Rest $R^1$ Methyl, Acetyl, Cyanomethyl, $\beta$-Aminoethyl oder Wasserstoff bezeichnet.

**3.** Polyalkylpiperidin-Derivate I nach Anspruch 1 oder 2, bei denen die Gruppe $R^2$ für einen Rest der Formel (a)

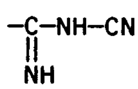 (a)

steht.

**4.** Polyalkylpiperidin-Derivate I nach Anspruch 1 oder 2, bei denen die Gruppe $R^2$ für einen Rest der Formel (b)

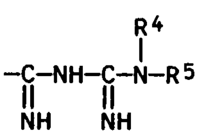 (b)

steht.

**5.** Polyalkylpiperidin-Derivate I nach Anspruch 1 oder 2, bei denen die Gruppe $R^2$ für einen Rest der Formel (c)

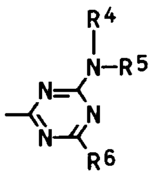

(c)

steht.

**6.** Polyalkylpiperidin-Derivate I nach den Ansprüchen 1 bis 5, bei denen der Rest $R^3$ Wasserstoff, geradkettiges oder verzweigtes $C_1$- bis $C_8$-Alkyl, Cyclohexyl, Benzyl, Phenyl oder bei Vorliegen einer durch $R^2$ bedingten Cyanguanidin-Struktur (a), einen Rest der Formel

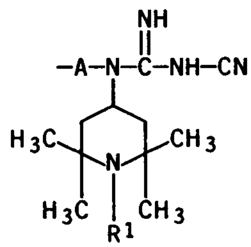

oder bei Vorliegen einer durch $R^2$ bedingten Biguanid-Struktur (b), einen Rest der Formel

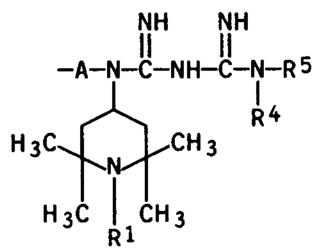

oder bei Vorliegen einer durch $R^2$ bedingten Triazin-Struktur (c), einen Rest der Formel

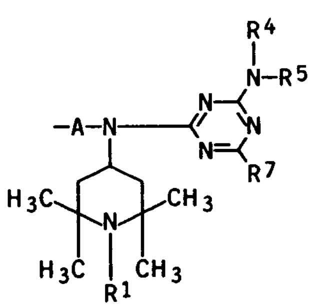

bedeutet.

**7.** Polyalkylpiperidin-Derivate I nach den Ansprüchen 1, 2, 4, 5 und 6, bei denen einer der Reste $R^4$ oder $R^5$ für Wasserstoff, geradkettiges oder verzweigtes $C_1$- bis $C_8$-Alkyl, Cyclohexyl oder Benzyl und der

andere für Phenyl, 4-$C_1$-$C_8$-Alkylphenyl, 4-$C_1$-$C_8$-Alkoxyphenyl, 4-Chlorphenyl, 4-Hydroxyphenyl oder 4-Cyanophenyl steht.

**8.** Polyalkylpiperidin-Derivate I nach den Ansprüchen 1, 2, 5, 6 und 7, bei denen der Rest $R^6$ Wasserstoff, geradkettiges oder verzweigtes $C_1$-bis $C_8$-Alkyl, Phenyl, Benzyl, Cyclohexyl oder einen Rest der Formel

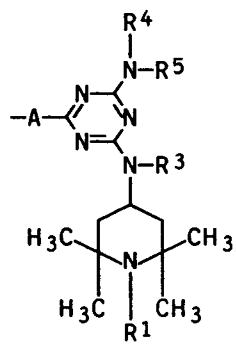

bezeichnet.

**9.** Gegen die Einwirkung von Licht, Sauerstoff und Wärme stabilisiertes organisches Material, enthaltend 0,01 bis 5 Gew.%, bezogen auf die Menge des organischen Materials, eines oder mehrerer Polyalkylpiperidin-Derivate I gemäß den Ansprüchen 1 bis 8, bei denen die Gruppe $R^2$ für einen Rest der Formel (a) oder (c) steht.

**10.** Verwendung von Polyalkylpiperidin-Derivaten I gemäß den Ansprüchen 1 bis 8, bei denen die Gruppe $R^2$ für einen Rest der Formel (a) oder (c) steht, als Lichtschutzmittel und Stabilisatoren für organisches Material.

**Patentansprüche für folgenden Vertragsstaat : ES**

**1.** Verfahren zur Herstellung von Polyalkylpiperidin-Derivaten der allgemeinen Formel I

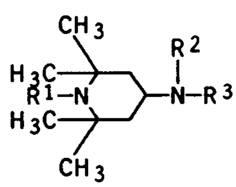

I

in der

$R^1$ Wasserstoff, $C_1$- bis $C_8$-Alkyl, Phenyl- oder Tolylalkyl mit 1 bis 4 C-Atomen in der Alkylgruppe, $C_1$- bis $C_6$-Acyl, Benzoyl, Allyl, Cyanmethyl, Hydroxyethyl, Aminoethyl, Hydroxyl oder das Oxyl-Radikal bezeichnet,

$R^2$ für einen Rest der Formel (a)

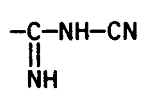

(a)

für einen Rest der Formel (b)

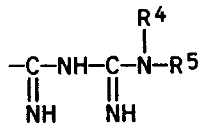

(b)

oder für einen Rest der Formel (c)

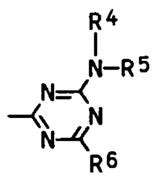

(c)

steht, wobei

R[4] und R[5] Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl, durch Ethersauerstoff- oder Stickstoff-Atome unterbrochenes $C_3$- bis $C_{22}$-Alkyl, welches zusätzlich Hydroxylgruppen tragen kann, Phenyl, das unsubstituiert oder ein- bis vierfach durch $C_1$- bis $C_{12}$-Alkyl, $C_3$- bis $C_{12}$-Cycloalkyl, $C_1$- bis $C_{12}$-Alkoxy, Chlor, Brom, $C_1$- bis $C_{12}$-Alkylamino, $C_1$- bis $C_{12}$-Dialkylamino, Phenyl, Benzyl, Hydroxyl oder Cyano substituiert sein kann, oder einen 5- oder 6-gliedrigen aromatischen Heterocyclus bedeuten, mit der Maßgabe, daß mindestens einer der Reste $R^4$ oder $R^5$ ein aromatischer Rest ist, und

R[6] Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis $C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl oder einen Rest der Formel

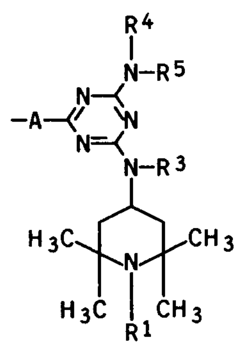

worin

A $C_2$- bis $C_{22}$-Alkylen, $C_8$- bis $C_{22}$-Cycloalkylen, $C_8$- bis $C_{16}$-Phenylalkylken, Phenylen oder durch Ethersauerstoff-Atome, Stickstoff-Atome oder 5- oder 6-gliedrige Heterocyclen unterbrochenes $C_4$- bis $C_{30}$-Alkylen bedeutet,

bezeichnet, und

$R^3$ Wasserstoff, $C_1$- bis $C_{22}$-Alkyl, $C_3$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Fluor, Chlor, Brom, $C_1$- bis $C_{12}$-Alkylamino oder $C_1$- bis $C_{12}$-Dialkylamino substituiert sein kann, $C_3$- bis $C_{12}$-Cycloalkyl oder Bicycloalkyl, durch Ethersauerstoff- oder Stickstoff-Atome unterbrochenes $C_4$- bis $C_{22}$-Alkyl, welches zusätzlich Hydroxylgruppen tragen kann, Phenyl, welches durch ein bis drei Methyl- oder Carbo-$C_1$-$C_{12}$-alkoxy-Gruppen substituiert sein kann, heterocyclische Reste enthaltendes $C_1$-bis $C_{22}$-Alkyl, einen Rest der Formel

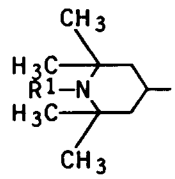

oder bei Vorliegen des Restes (a) für $R^2$ einen Rest der Formel

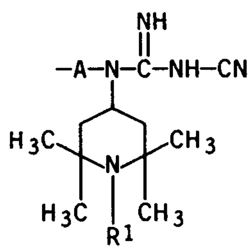

oder bei Vorliegen des Restes (b) für $R^2$ einen Rest der Formel

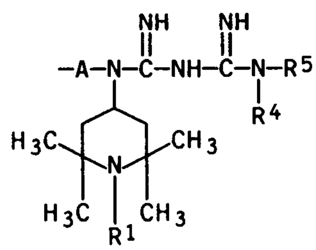

oder bei Vorliegen des Restes (c) für $R^2$ einen Rest der Formel

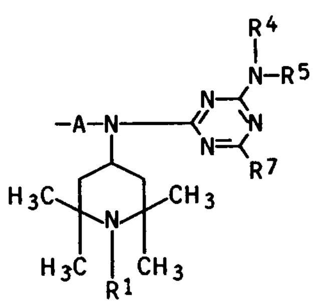

wobei $R^7$ Wasserstoff, $C_1$- bis $C_{22}$-Alkyl, $C_2$- bis $C_{22}$-Alkenyl, $C_7$- bis $C_{22}$-Phenyl- und Diphenylalkyl, wobei der Phenylkern unsubstituiert oder ein- bis dreifach durch $C_1$- bis $C_{12}$-Alkyl, $C_1$- bis $C_{12}$-Alkoxy, Chlor, Brom, $C_1$- bis $C_{12}$-Alkylamino, $C_1$- bis $C_{12}$-Dialkylamino, Hydroxyl, Cyano oder $C_1$- bis $C_{12}$-Carboalkoxy substituiert sein kann, Phenyl, das unsubstituiert oder ein- bis dreifach durch $C_1$- bis $C_{12}$-Alkyl, $C_1$- bis

$C_{12}$-Alkoxy, Chlor, Brom, Phenyl, $C_1$- bis $C_{12}$-Alkylamino, $C_1$- bis $C_{12}$-Dialkylamino, Hydroxyl, Cyano, $C_1$- bis $C_{12}$-Carbonyloxy oder $C_1$- bis $C_{12}$-Carboalkoxy substituiert sein kann, $C_3$- bis $C_{12}$-Cycloalkyl, Pyridyl oder durch Carbonyloxy unterbrochenes $C_2$- bis $C_{22}$-Alkyl bedeutet,

bezeichnet,

sowie Tautomeren und Säureadditionssalzen der Verbindungen I, dadurch gekennzeichnet, daß man Amine der allgemeinen Formel III oder IIIa

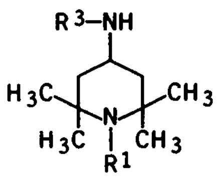

III

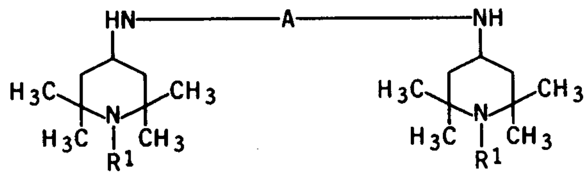

IIIa

in denen die Variablen $R^1$, $R^3$ und A die oben genannten Bedeutungen haben, mit Natriumdicyanamid zu den Polyalkylpiperidin-Derivaten mit Cyanguanidin-Gruppierungen Ia umsetzt,

zum Erhalt der Biguanid-Derivate Ib die Cyanguanidine Ia mit Aminen der allgemeinen Formel $HNR^4R^5$, in der die Variablen $R^4$ und $R^5$ die oben genannten Bedeutungen haben, umsetzt,

zum Erhalt der Polyalkylpiperidin-Derivate mit Trizian-Ringen Ic die Biguanid-Derivate Ib mit Carbonsäuren oder Carbonsäure-Derivaten der allgemeinen Formeln $R^6$-COX, $R^6$-CN, XOC-A-COX, NC-A-COX oder NC-A-CN, wobei X für Hydroxyl, Chlor, Brom oder $C_1$- bis $C_4$-Alkoxy steht und die Variablen $R^6$ und A die oben genannten Bedeutungen haben, umsetzt, und

zum Erhalt der Säureadditionssalze der Verbindungen I diese mit geeigneten Säuren umsetzt.

**2.** Verfahren nach Anspruch 1, wobei der Rest $R^1$ Methyl, Acetyl, Cyanomethyl, $\beta$-Aminoethyl oder Wasserstoff bezeichnet.

**3.** Verfahren nach Anspruch 1 oder 2, wobei die Gruppe $R^2$ für einen Rest der Formel (a)

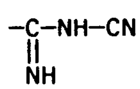

(a)

steht.

**4.** Verfahren nach Anspruch 1 oder 2, wobei die Gruppe $R^2$ für einen Rest der Formel (b)

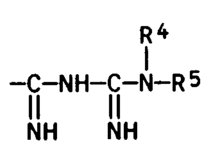

(b)

steht.

**5.** Verfahren nach den Ansprüchen 1 bis 5, wobei der Rest $R^3$ Wasserstoff, geradkettiges oder verzweigtes $C_1$- bis $C_8$-Alkyl, Cyclohexyl, Benzyl, Phenyl oder bei Vorliegen einer durch $R^2$ bedingten

Cyanguanidin-Struktur (a), einen Rest der Formel

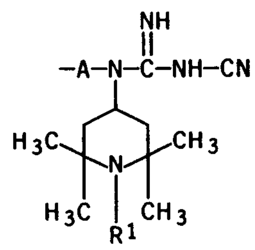

oder bei Vorliegen einer durch $R^2$ bedingten Biguanid-Struktur (b), einen Rest der Formel

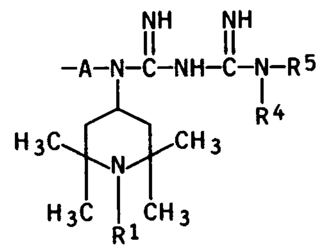

oder bei Vorliegen einer durch $R^2$ bedingten Triazin-Struktur (c), einen Rest der Formel

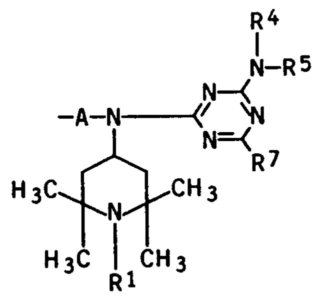

bedeutet.

**6.** Verfahren nach den Ansprüchen 1, 2, 4 und 5, wobei einer der Reste $R^4$ oder $R^5$ für Wasserstoff, geradkettiges oder verzweigtes $C_1$- bis $C_8$-Alkyl, Cyclohexyl oder Benzyl und der andere für Phenyl, 4-$C_1$-$C_8$-Alkylphenyl, 4-$C_1$-$C_8$-Alkoxyphenyl, 4-Chlorphenyl, 4-Hydroxyphenyl oder 4-Cyanophenyl steht.

**7.** Verfahren nach den Ansprüchen 1, 2, 5 und 6, wobei der Rest $R^6$ Wasserstoff, geradkettiges oder verzweigtes $C_1$-bis $C_8$-Alkyl, Phenyl, Benzyl, Cyclohexyl oder einen Rest der Formel

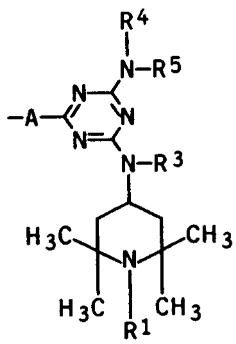

bezeichnet.

**8.** Verfahren zum Stabilisieren von organischem Material gegen die Einwirkung von Licht, Sauerstoff und Wärme, dadurch gekennzeichnet, daß man hierzu Polyalkylpiperidin-Derivate I gemäß den Ansprüchen 1 bis 7, bei denen die Gruppe $R^2$ für einen Rest der Formel (a) oder (c) steht, einsetzt.

**9.** Verfahren nach Anspruch 8, dadurch gekennzeichnet, daß man hierbei 0,01 bis 5 Gew.%, bezogen auf die Menge des organischen Materials, eines oder mehrerer Polyalkylpiperidin-Derivate I gemäß den Ansprüchen 1 bis 7 bei denen die Gruppe $R^2$ für einen Rest der Formel (a) oder (c) steht, einsetzt.